(19) Europäisches Patentamt
European Patent Office
Office européen des brevets

(11) Numéro de publication: **0 186 052**
**A1**

# (12) DEMANDE DE BREVET EUROPEEN

(21) Numéro de dépôt: **85115809.7**

(22) Date de dépôt: **11.12.85**

(51) Int. Cl.⁴: **A 01 N 59/14**
A 01 N 59/00, C 11 D 3/39
//(A01N59/14, 37:02),
(A01N59/00, 37:02)

(30) Priorité: **12.12.84 FR 8419109**

(43) Date de publication de la demande:
**02.07.86 Bulletin 86/27**

(84) Etats contractants désignés:
**AT BE CH DE FR GB IT LI NL SE**

(71) Demandeur: **INTEROX Société anonyme dite:**
**Rue du Prince Albert, 33**
**B-1050 Bruxelles(BE)**

(72) Inventeur: **Walraevens, René**
**Avenue des Neuf Provinces, 3**
**B-1080 Bruxelles(BE)**

(72) Inventeur: **Delplanque-Janssens, Francine**
**Immelvoortstraat 19**
**B-1850 Grimbergen(BE)**

(74) Mandataire: **Lederer, Franz, Dr. et al,**
**Patentanwälte Dr. Franz Lederer Dipl.-Ing. Reiner F. Meyer-Roxlau Lucile-Grahn-Strasse 22**
**D-8000 München 80(DE)**

## (54) Procédé d'activation du peroxyde d'hydrogène dans des bains de lavage ou de désinfection.

(57) Procédé d'activation du peroxyde d'hydrogène dans des bains de lavage et de désinfection mettant en oeuvre un activant de formule:

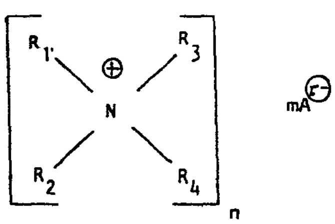

dans laquelle,

$R_1$, $R_2$ et $R_3$ sont des groupes alkyle ou aryle éventuellement substitués, $R_4$ est un groupe alkyle ou aryle substitué par au moins un groupement ester, m, r et n sont des nombres entiers et $A^{r-}$ est un anion tel que m x r = n. L'activant peut être incorporé à une poudre à lessiver les textiles ou à récurer les surfaces dures.

EP 0 186 052 A1

TITRE MODIFIÉ
voir page de garde



**Procédé d'activation du peroxyde d'hydrogène dans des bains de lavage ou de désinfection, compositions solides de lavage et de désinfection et utilisation de telles composition dans des bains pour le lavage ou la désinfection des textiles.**

Cas INT.84/2

INTEROX (Société Anonyme)

La présente invention concerne un procédé d'activation du peroxyde d'hydrogène. Elle concerne plus particulièrement l'activation du peroxyde d'hydrogène ou des composés libérant du peroxyde d'hydrogène dans les bains de lavage et de désinfection pour en augmenter l'efficacité à basse température. Elle concerne aussi des compositions solides de lavage ou de désinfection contenant un composé capable de libérer du peroxyde d'hydrogène en solution aqueuse et un activant du peroxyde d'hydrogène.

Les composés peroxydés sont utilisés depuis de nombreuses années dans les compositions de lavage ou de désinfection pour leur action blanchissante ou désinfectante résultant de leurs propriétés oxydantes. On les utilise habituellement sous la forme de persels de métaux alcalins tels que par exemple, les perborates de sodium tétrahydraté et monohydraté et le percarbonate de sodium.

Les persels libèrent dans le milieu aqueux de lavage du peroxyde d'hydrogène dont l'efficacité oxydante est maximale à des températures qui dépassent 80°C. Cependant, depuis plusieurs années, on assiste à un accroissement de l'usage des textiles synthétiques qu'il n'est généralement pas indiqué de soumettre à des températures de lavage supérieures à 60°C. D'autre part, l'accroissement important du coût de l'énergie a entraîné une tendance croissante pour le lavage à des températures plus basses allant de l'ambiance à 60°C au maximum.

Pour rendre le peroxyde d'hydrogène des bains aqueux de lavage ou de désinfection efficace à d'aussi basse températures, il a été proposé d'ajouter aux bains des activants capables de libérer un

peracide par perhydrolyse au moyen des ions perhydroxyles ($HOO^-$) générés par la dissociation du peroxyde d'hydrogène dans le bain.

A titre d'activants, on a notamment proposé les esters des acides carboxyliques (brevet US-A-2 448 252) (E.I. du PONT de NEMOURS) * revendication 1; colonne 4 lignes 2 à 4 *; brevet US-A-2 955 905 (LEVER BROTHERS Cy) * revendications 6 à 18, colonne 3 lignes 3 et 4 et colonne 4 lignes 14 à 21 *).

On a aussi proposé les composés d'ammonium quaternaire comprenant les groupements ester phénoxy (brevet GB-B-1 382 594 (UNILEVER LIMITED)).

Ces derniers activants présentent la particularité que le peracide généré par la réaction de perhydrolyse est adsorbé sur la surface des fibres, ce qui risque lorsqu'ils sont employés dans des compositions de lavage du linge de donner lieu à des phénomènes de dégradation localisée du textile ainsi qu'à un blanchiment non uniforme des fibres voire même dans certains cas à une dégradation partielle et irrégulière des colorants imprégnés dans les fibres.

L'invention remédie à ces inconvénients en fournissant un procédé d'activation du peroxyde d'hydrogène dans des bains aqueux, qui présente une grande efficacité, sans nuire aux propriétés des fibres ou des textiles mis en contact avec ces bains.

L'invention concerne à cet effet un procédé d'activation du peroxyde d'hydrogène dans les bains de lavage et de désinfection par incorporation au bain d'un activant du peroxyde d'hydrogène; selon l'invention l'activant est sélectionné parmi les composés d'ammonium quaternaire de formule générale :

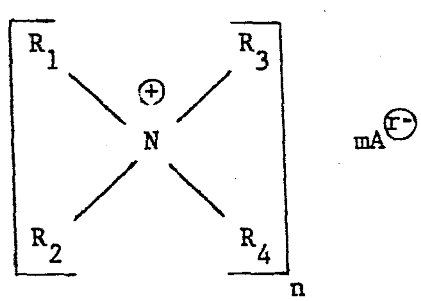

dans laquelle,

$R_1$, $R_2$ et $R_3$ représentent des groupes alkyle, aryle, alkylaryle ou arylalkyle éventuellement substitués, $R_4$ représente un groupe alkyle, aryle, alkylaryle ou arylalkyle substitué par au moins un groupement ester, $A^{\ominus}$ représente un anion organique ou inorganique, n et r sont des nombres entiers et m est un nombre entier valant 1, 2 ou 3 et tel que le produit m x r = n.

Selon l'invention, dans la formule générale précitée, les groupes alkyle sont toutes chaînes aliphatiques ramifiées ou non, tous radicaux cycloalkyles ou toutes chaînes aliphatiques substituées par un radical cycloalkyle éventuellement substitué tel que les chaînes droites ou ramifiées de 1 à 20 atomes de carbone comme les groupes méthyle, propyle, isopropyle, n-butyle, isobutyle, sec-butyle et tert-butyle, n-pentyle, 1-méthylbutyle, 2-méthylbutyle, 3-méthylbutyle, 1-éthylpropyle, 1,1-diméthylpropyle, 2,2-diméthylpropyle, n-hexyle et ses isomères ramifiés, n-heptyle, n-octyle, n-nonyle, n-décyle, n-undécyle, n-dodécyle et leurs isomères ramifiés, les groupes cyclopropyle, cyclopentyle et cyclohexyle et les groupes cités comportant une ou plusieurs insaturations.

Les groupes aryle sont tout dérivé aromatique mono ou polycyclique éventuellement substitué tel que les groupes phényle, 2,3-xylyle, 2,4-xylyle, 2,5-xylyle, 2-tolyle, 3-tolyle, 4-tolyle, mésityle, o-cuményle, m-cuményle, p-cuményle, 1-naphtyle, 2-naphtyle, 1-anthryle, 2-anthryle, 1-phénantryle, 2-phénantryle, 2-chrysyle et 2-pyryle.

Les groupes alkylaryle sont tout groupe aryle substitué par un ou plusieurs groupes alkyle saturés ou insaturés tel que les groupes alkylbenzyle, alkylnaphtyle et alkylanthryle.

Les groupes arylalkyle sont tous groupes alkyle substitués par un ou plusieurs groupes aryle tels que les groupes benzyle, phénétyle et trityle.

Par anion inorganique ou organique on entend tout anion provenant d'un acide inorganique ou organique tel que les anions chlorure, bromure, iodure, fluorure, sulfate, hydrogénosulfate, carbonate, bicarbonate, phosphate, monohydrogénophosphate, dihydrogénophosphate, pyrophosphate, métaphosphate, thiosulfate, nitrate,

méthosulfate, dodécylsulfate, dodécylbenzènesulfonate, phosphonate, méthylphosphonate, methanedisulfonate, méthylsulfonate, éthanesulfonate, 1,2-éthanedisulfonate.

Selon l'invention, le groupe $R_4$ est substitué par au moins un groupement ester. Celui-ci peut dériver de tout acide mono ou polycarboxylique tel que les acides acétique, propionique, butanoïque, octanoïque, nonanoïque et décanoïque. Les composés dans lesquels les groupements ester aliphatique sont à chaîne droite ou ramifiée comprenant de 8 à 10 atomes de carbone conviennent bien, tels que le chlorure de méthyl-tris(2-octyloxyéthyl)-ammonium.

On préfère les composés dans lesquels le groupe $R_4$ est substitué par au moins deux groupements esters, tel que, par exemple, le chlorure de triméthyl-(2,3-diacétoxypropyl)-ammonium.

Les groupes $R_1$, $R_2$ et $R_3$ peuvent être des groupes non substitués ou des groupes substitués par des groupements fonctionnels organiques tels que les groupements alcool, carbonyle, carboxyle, amine, amide, nitrile, éther ou ester.

Des composés préférés dans l'exécution du procédé selon l'invention sont ceux dans lesquels l'un au moins des groupes $R_1$, $R_2$ et $R_3$, de préférence chacun de ces groupes est substitué par au moins un groupement ester comme défini plus haut. Parmi ces composés, on peut notamment citer ceux dans lesquels l'un au moins des groupements ester est un groupement ester acétique, tels que, par exemple, le chlorure de n-butyl-tris(2,3-diacétoxypropyl)-ammonium.

Des composés qui se sont avérés avantageux dans la mise en oeuvre du procédé selon l'invention sont les sels de trialkyl-(2,3-diacétoxypropyl)-ammonium, les sels d'alkyl-tris(2-acétoxyéthyl)-ammonium, les sels d'alkyl-tris(2,3-diacétoxypropyl)-ammonium, les sels de tétrakis(2-acétoxyéthyl)-ammonium et les sels de (2,3-diacétoxypropyl)-tris-(2-acétoxyéthyl)-ammonium. Des exemples de tels sels, utilisables dans le cadre de l'invention sont le méthosulfate de méthyl-tris(2-acétoxyéthyl)-ammonium, le dodécylsulfate de méthyl-tris(2-acétoxyéthyl)-ammonium, le dodécylbenzènesulfonate de méthyl-tris(2-acétoxyéthyl)-ammonium, le chlorure de méthyl-tris (2-acétoxyéthyl)-ammonium, le bromure de tétrakis(2-acétoxyéthyl)-ammonium et le chlorure de tris(2-acétoxyéthyl)-(2,3-diacétoxypropyl)-ammonium.

Dans le procédé selon l'invention, le peroxyde d'hydrogène peut être incorporé tel quel au bain de lavage, ou formé in situ dans le bain, par dissolution, dans celui-ci, d'un composé capable de générer du peroxyde d'hydrogène en solution aqueuse. Ce composé peut être choisi parmi les peroxydes métalliques notamment, les peroxydes de métaux alcalins ou alcalino-terreux (par exemple le peroxyde de sodium) et les persels inorganiques tels que les perborates, les percarbonates et les persulfates. Les persels préférés sont les perborates et les percarbonates des métaux alcalins.

Dans le procédé selon l'invention, la quantité d'activant mis en oeuvre doit être suffisante pour réagir avec une quantité substantielle des ions perhydroxyles du bain. La quantité optimum d'activant dépend d'un certain nombre de paramètres tels que la température de lavage, le nombre de groupements ester par mole d'activant et la cinétique de la perhydrolyse de l'activant. On utilise généralement des quantités d'au moins 0,05 mole par atome-gramme d'oxygène actif du bain. Des quantités préférées sont celles comprises entre 0,25 et 1,5 mole d'activant par atome-gramme d'oxygène actif.

La température du bain doit être inférieure à son point d'ébullition. Elle est de préférence inférieure à 75°C; les températures comprises entre 15 et 60°C étant spécialement avantageuses.

L'invention concerne aussi des compositions solides de lavage ou de désinfection comprenant un composé susceptible de libérer du peroxyde d'hydrogène en solution aqueuse et un activant conforme à celui utilisé dans le procédé selon l'invention.

Dans les compositions selon l'invention, le composé capable de libérer du peroxyde d'hydrogène en solution aqueuse peut être tout composé solide hydrosoluble, qui est normalement stable aux températures normales de stockage et de manutention des compositions et qui libère du peroxyde d'hydrogène lorsqu'il est dissous dans de l'eau. Il peut par exemple être choisi parmi les peroxydes métalliques décrits plus haut.

Les compositions selon l'invention sont généralement à l'état de poudres. Elles peuvent convenir pour divers usages tels que le lavage des textiles industriels ou ménagers, le blanchiment des textiles, le récurage des surfaces dures, la désinfection du linge. Leur teneur en composé capable de libérer du peroxyde d'hydrogène dépend du type d'application à laquelle elles sont destinées. Dans le cas de poudres à lessiver ou à récurer, cette teneur peut varier par exemple entre 2 et 50 % en poids de la composition, de préférence entre 8 et 30 %. Dans le cas de poudres à blanchir elle peut être comprise entre 40 et 99 % du poids de la composition, de préférence entre 60 et 95 % de ce poids.

Les compositions solides selon l'invention peuvent contenir généralement, outre le composé capable de libérer du peroxyde d'hydrogène et l'activant, des substances tensio-actives, des builders de détergence et d'autres additifs communément employés dans les formulations détergentes.

On peut utiliser des substances tensio-actives, anioniques, cationiques, non-ioniques ou amphotères compatibles avec les activants utilisés. Des exemples de substances tensio-actives sont les alkylbenzènesulfonates, les alpha-oléfines sulfonates, les sulfates d'alcool et d'alcool éthoxylé, les polyoxyéthylènes, les alcools éthoxylés, les esters de polyéthylène glycol les oxydes d'amine, les alkyléthoxylates d'amine, les sels d'ammonium quaternaire et les dérivés amphotères de l'imidazolinium. D'autres exemples de substances tensio-actives sont donnés dans le traité KIRK-OTHMER Encyclopedia of Chemical Technology 3e édition, volume 22 : "Sulfonation and Sulfation to Thorium and Thorium Compounds" 1983, JOHN WILEY AND SONS, Inc, New-York, pages 332 à 432
* Surfactants, pages 332 à 387 *

Les builders de détergence sont choisis parmi les substances qui augmentent l'effet détersif des tensio-actifs telles que les phosphates inorganiques traditionnellement utilisés pour leurs propriétés séquestrantes des ions métalliques et pour leurs propriétés dispersantes des particules de salissures dans la liqueur de lavage. Des exemples de phosphates le plus souvent employés sont

le tripolyphosphate pentasodique, le pyrophosphate tétrasodique, l'orthophosphate trisodique, l'hexamétaphosphate de sodium et les polymétaphosphates de sodium. D'autres sels inorganiques peuvent aussi être utilisés seuls ou en combinaison avec des phosphates à savoir le carbonate et le bicarbonate de sodium, les silicates de sodium et le borax.

D'autres exemples de builders utilisables dans les compositions selon l'invention sont les aminocarboxylates comme le nitrilotriacétate trisodique et l'éthylènediaminetétraacétate tétrasodique, les zéolites, les aminophosphonates, les polyacrylates tels que les acides polyacryliques. Parmi ces derniers, les polyhydroxyacrylates donnent de bons résultats. Des composés préférés sont le sel de sodium et la polylactone de l'acide poly-alpha-hydroxyacrylique.

D'autres additifs peuvent aussi être incorporés aux compositions solides selon l'invention en fonction du domaine d'application auquel elles sont destinées. Parmi ces additifs, on peut mentionner les azurants optiques, les agents anti-mousse et régulateurs de mousse, les enzymes, les agents anti-redéposition, les inhibiteurs de corrosion, les parfums, les colorants, les agents régulateurs de pH et les matières de charge.

Des azurants optiques utilisables sont par exemple les agents fluorescents dérivés du stilbène comme les bistriazinylstilbènes et les mono et bis(azol-2-yl)stilbènes.

Des exemples d'agents anti-mousse et régulateurs de mousse sont les silicones et particulièrement les cires micro-cristallines et les silicones auto-émulsionnants tels que les copolymères siloxane-glycol. D'autres agents régulateurs de mousse convenant bien sont les savons d'acides gras, les mélanges de savons et tensio-actifs non-ioniques et les esters d'alkyl phosphate tel que les phosphates de mono stéaryle, de di et de tristéaryle et les phosphates d'oléyle.

Les enzymes pouvant être introduites dans les compositions selon l'invention ont pour fonction de faciliter l'enlèvement de taches à base de sang et de protéines. Elles sont généralement choisies dans la classe des protéases.

Les agents anti-redéposition servent à exercer une action de mise en suspension des particules de salissure dans le bain de lavage et partant, évitent qu'elles n'aillent se fixer sur les plages non souillées de la matière à laver. Des exemples d'agents anti-redéposition sont la carboxyméthylcellulose de sodium et les autres alkylcelluloses telles que la méthylcellulose, l'hydroxybutylcellulose et l'hydroxypropylcellulose. D'autres produits tels que la polyvinylpyrrolidone et l'alcool polyvinylique peuvent aussi être employés.

Les inhibiteurs de corrosion ont pour fonction de réduire substantiellement la dégradation des matériaux en contact avec les bains de lavage. Ils sont choisis parmi les composés inorganiques ou organiques. Des exemples de ceux-ci sont les polyphosphates, les silicates ou les borates, les acides benzoïques éventuellement substitués et l'acide benzène sulfonique.

Les compositions selon l'invention peuvent éventuellement contenir aussi des colorants dont la fonction est de mettre en évidence ou de contrôler la présence de certains de ses constituants. Des exemples de colorants utilisables sont ceux admis par la "Food and Drug Association" aux Etats-Unis, dans la catégorie "Drugs and Cosmetics", tels que, par exemple, les colorants nitrés (Jaune N°7), azoïques (Rouge N°17), anthraquinoniques (Violet N°2 et Vert N°5 et 6) et dérivés de l'indigo (Bleu N°2 et 6).

Les parfums servent à conférer une odeur agréable et stable aux compositions de lavage, aux bains de lavage et aux textiles traités. Ils doivent en général présenter une bonne stabilité chimique vis-à-vis des autres constituants des compositions et ne donner lieu qu'à une faible diffusion à travers l'emballage. Comme exemples, on peut citer le lyral, le tétrahydrocitral et le dihydromyrcénol.

Les agents régulateurs de pH comprennent des agents alcalins et des mélanges tampon comme les carbonates, bicarbonates phosphates et silicates des métaux alcalins.

Les matières de charge sont généralement des sels inorganiques inertes; les plus courantes sont le sulfate de sodium et le chlorure de sodium. Le sulfate de sodium est habituellement préféré.

L'invention concerne aussi l'utilisation des compositions selon l'invention dans des bains pour le lavage, le blanchiment et la désinfection de textiles. Dans cette utilisation, les compositions, selon l'invention doivent être mises en oeuvre en quantité réglée pour assurer un traitement efficace et économique des textiles. On choisit par exemple des teneurs allant de 0,5 à 10 g/l de bain de lavage et, de préférence 0,7 à 5 g/l.

La durée du traitement de lavage, blanchiment ou désinfection peut généralement être inférieure à 90 min.; elle est de préférence comprise entre 4 et 60 min.

Les exemples qui suivent sont fournis pour illustrer l'invention.

Les exemples concernent les activants suivants, qui sont conformes à ceux mis en oeuvre dans le procédé et les compositions selon l'invention.

TMAEACl : chlorure de triméthyl-(2-acétoxyéthyl)-ammonium;
TMAPACl : chlorure de triméthyl-(2-acétoxypropyl)-ammonium;
MTAEAMS : méthosulfate de méthyl-tris (2-acétoxyéthyl)-ammonium;
MTAEADS : dodécylsulfate de méthyl-tris(2-acétoxyéthyl)-ammonium;
MTAEADBS : dodécylbenzènesulfonate de méthyl-tris(2-acétoxyéthyl)-ammonium;
MTAEACl : chlorure de méthyl-tris(2-acétoxyéthyl)-ammonium;
TAEABr : bromure de tétrakis(2-acétoxyéthyl)-ammonium;
TMDAPACl : chlorure de triméthyl-(2,3-diacétoxypropyl)-ammonium;
TEDAPACl : chlorure de triéthyl-(2,3-diacétoxypropyl)-ammonium;
TAEDAPCl : chlorure de (2,3-diacétoxypropyl)-tris(2-acétoxyéthyl)-ammonium;
BTDAPACl : chlorure de n-butyl-tris(2,3-diacétoxypropyl)-ammonium;

Première série d'exemples

Les exemples 1 à 13 qui vont suivre concernent des essais au cours duquel on a mesuré l'aptitude des activants précités à générer des peracides dans des bains aqueux. A cet effet, on a procédé de la manière suivante.

Dans un des compartiments d'une machine à laver de laboratoire de marque TERGOTOMETER fabriquée par U.S. Testing Co (Hoboken,

New-York, USA), on a introduit 1 litre d'eau déminéralisée à 40°C. On a réglé le thermostat de la machine à 40°C et la vitesse d'agitation à 90 tours/min. On a ensuite introduit immédiatement après avoir enclenché un chronomètre, 5 g d'une poudre à lessiver standardisée par la Commission Electrotechnique Internationale (CEI), composée de :

| | g/100 g |
|---|---|
| Sel de sodium d'alkylbenzènesulfonate linéaire (longueur moyenne de la chaîne alkyle : 11,5 carbones) | 8,00 |
| Alcool de suif éthoxylé (14 unités oxyde d'éthylène) | 2,87 |
| Savon de sodium (chaîne en $C_{12}$-$C_{16}$ : 13 à 26 %, chaîne en $C_{18}$-$C_{22}$ : 74 à 87 %) | 3,50 |
| Tripolyphosphate de sodium | 43,75 |
| Silicate de sodium (rapport $SiO_2$ : $Na_2O$ = 3,3) | 7,50 |
| Silicate de magnésium | 1,88 |
| Carboxyméthylcellulose | 1,25 |
| Ethylènediaminetétraacétate de sodium | 0,25 |
| Azurant optique pour coton (de type stilbénique) | 0,25 |
| Sulfate de sodium | 21,00 |
| Eau | 9,75 |
| | 100,00 |

Après 30 s d'agitation on a introduit simultanément 1 g de perborate de sodium tétrahydraté (6,5 mmol) et 1,3 mmol d'activant (20 % de la quantité de perborate) et on a ajusté si nécessaire le pH à 10 au moyen d'une solution de soude caustique 2N.

On a prélevé ensuite, à intervalles réguliers, des échantillons d'environ 50 ml de solution pour le dosage du peracide forme. Chacun de ces échantillons a été introduit immédiatement après son prélèvement, dans un erlenmeyer de 250 ml préalablement taré contenant de la glace déminéralisée pilée et 10 ml d'un tampon acétique constitué de 12 % en volume d'acide acétique glacial, 38 % d'eau déminéralisée et 50 % d'une solution de soude caustique 2N . La différence de poids de l'erlenmeyer après introduction de l'échantillon fournit le poids de celui-ci. Après avoir agité le

contenu de l'erlenmeyer et s'être assuré que la température de la solution est inférieure à 5°C on a ajouté 10 ml d'une solution N d'iodure de potassium. On a titré ensuite jusqu'à décoloration de la solution et le plus rapidement possible l'iode libéré par le peracide au moyen d'une solution 0,02 N de thiosulfate de sodium. L'échantillon qui contient le plus de peracide est choisi pour fournir le résultat du test de perhydrolyse. Ce dernier est exprimé en mol peracide/mol activant et en mol peracide/g activant.

Les résultats des essais ont été consignés au tableau 1. Ils illustrent la capacité de génération de peracide dans le milieu et les conditions de lavage par les divers activants cités plus haut.

Tableau I

| Exemple N° | Activant | mol peracide/mol activant | mmol peracide/g activant |
|---|---|---|---|
| 1 | TMAEACl | 0,47 | 2,59 |
| 2 | TMAPACl | 0,22 | 1,21 |
| 3 | MTAEAMS | 1,40 | 3,49 |
| 4 | MTAEADS | 1,40 | 2,51 |
| 5 | MTAEDBS | 1,40 | 2,27 |
| 6 | MTAEACl | 1,40 | 4,30 |
| 7 | TAEABr | 1,60 | 3,62 |
| 8 | TMDAPACl | 1,00 | 3,94 |
| 9 | TEDAPACl | 0,82 | 2,77 |
| 10 | TAEDAPCl | 2,50 | 5,32 |
| 11 | BTDAPACl | 1,90 | 3,25 |

Deuxième série d'exemples

Les exemples 14 et 15 qui vont suivre concernent des essais destinés à mesurer la cinétique de la génération de peracide dans des bains de lavage contenant des compositions de lavage conformes à l'invention. Les essais ont été réalisés dans la machine de laboratoire TERGOTOMETER comme pour les exemples 1 à 13 et avec la même poudre à lessiver CEI. L'eau employée titrait 20°C hydroti-

métriques français de dureté. La composition du bain a été :

| | |
|---|---|
| Poudre CEI | 5g/l |
| Perborate de sodium tétrahydraté | 1g/l |
| TMDAPACl cristallisé avec 0,5 $H_2O$ | 0,341g/l |

L'activant TMDAPACl a été mis en oeuvre en quantité réglée pour activer 20 % du perborate de sodium présent et on a mesuré l'évolution dans le temps de la concentration du bain en acide peracétique ainsi que du pH par prélèvements de parties aliquotes et détermination de la teneur en $CH_3-CO_3H$.

Exemple 14

Dans cet exemple, on a mis en oeuvre un bain de lavage exempt de salissures, on a maintenu sa température à 40°C et on a ajusté son pH à environ 10, au moyen d'une solution 2N d'hydroxyde de sodium.

Les résultats ont été portés au Tableau II. Ils montrent que l'activation du perborate par le TMDAPACl donne lieu à une génération rapide d'acide peracétique dans le milieu de lavage.

Tableau II

| Durée (min.) | $CH_3-CO_3H$ (mmol/l) | pH |
|---|---|---|
| 0 | 0 | 10,0 |
| 5 | 1,2 | 9,8 |
| 10 | 1,3 | 9,7 |
| 15 | 1,25 | 9,7 |

Exemple 15

On a répété l'essai de l'exemple 14, avec un bain dont le pH a été ajusté à environ 9. Les résultats sont repris au Tableau III.

Tableau III

| Durée (min.) | $CH_3-CO_3H$ (mmol/l) | pH |
|---|---|---|
| 0 | 0 | 9,0 |
| 5 | 0,72 | 9,0 |
| 10 | 0,96 | 8,9 |
| 15 | 1,12 | 8,9 |

Les exemples 14 et 15 montrent que la concentration maximum en acide peracétique dans le bain est atteinte endéans les temps courts de 10 à 15 minutes selon le pH de départ.

Troisième série d'exemples

Les exemples 16 à 19 concernent des essais visant à montrer l'influence du procédé selon l'invention sur l'efficacité du lavage des textiles. Dans ces essais, les salissures ont été mises en oeuvre, imprégnées sur des épouvettes de tissus en polyester-coton. Trois types de salissures ont été employés : le thé, le vin rouge, et le jus de myrtilles. On a mesuré après lavage le taux de leur décoloration en vue d'évaluer l'activité blanchissante du bain de lavage.

Les essais ont été réalisés dans la machine de laboratoire TERGOTOMETER sous une agitation réglée à 90 tours/min.

On a mis en oeuvre le même bain de lavage qu'à l'exemple 14.

L'appréciation de la décoloration des éprouvettes de tissus s'est faite par mesure de leur réflectance avant et après lavage au moyen d'un photocolorimètre de marque ZEISS (type RFC 3) équipé d'une source lumineuse simulant la lumière du jour D 6500 et d'un filtre vert FMY/L normalisé par la Commission Internationale de l'Eclairage.

Le taux de salissure éliminée par lavage a été calculé à partir des mesures de réflectance au moyen de la formule suivante :

$$SE = \frac{Rl - Rt}{Ro - Rt} \times 100 \qquad \text{où :}$$

SE : taux de salissure éliminée, en %

Rl : réflectance du tissu après lavage

Rt : réflectance du tissu taché avant lavage

Ro : réflectance du tissu non taché

Exemples 16 et 17 (conformes à l'invention)

On a mis en oeuvre le même bain de lavage qu'à l'exemple 14 et la température de lavage a été fixée à 40°C (exemple 16) et 60°C (exemple 17).

Les essais de lavage ont duré 20 minutes et se sont déroulés selon le mode opératoire suivant :

- au temps 0, introduction de la poudre CEI dans l'eau préchauffée à la température de l'essai;
- après 30 s, introduction du perborate et de l'activant;
- de 30 à 60 s, ajustement du pH à 10;
- après 60 s, introduction des éprouvettes de tissus tachés;
- après 21 min. enlèvement des éprouvettes de tissus, rinçage à l'eau froide de même dureté suivi d'un essorage et d'un séchage à l'air ambiant.

Les résultats obtenus ont été repris au Tableau IV.

Tableau IV

| Exemple N° | Température °C | Activant | Efficacité (SE, %) | | |
|---|---|---|---|---|---|
| | | | Thé | Vin rouge | myrtilles |
| 16 | 40 | TMDAPACl | 36 | 25 | 51 |
| 17 | 60 | TMDAPACl | 28 | 28 | 66 |

Exemples 18 et 19 (exemples de référence)

On a répété les essais des exemples 16 et 17, en omettant d'introduire l'activant (TMDAPACl) dans le bain. Les résultats des essais sont repris au Tableau V.

Tableau V

| Exemple N° | Température °C | Activant | Efficacité (SE, %) | | |
|---|---|---|---|---|---|
| | | | Thé | Vin rouge | myrtilles |
| 18 | 40 | néant | 18 | 16 | 41 |
| 19 | 60 | néant | 24 | 19 | 61 |

Une comparaison des résultats des exemples 16 et 17 avec ceux des exemples 18 et 19 montrent l'avantage considérable apporté par l'activant sélectionné tant à 60°C qu'à 40°C.

## R E V E N D I C A T I O N S

1 - Procédé d'activation du peroxyde d'hydrogène dans les bains de lavage et de désinfection selon lequel on incorpore au bain un activant du peroxyde d'hydrogène caractérisé en ce qu'on sélectionne l'activant parmi les composés d'ammonium quaternaire de formule générale :

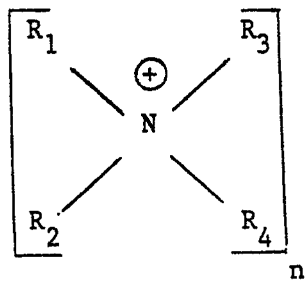

$mA^{r\ominus}$ dans laquelle,

$R_1$, $R_2$ et $R_3$ représentent des groupes alkyle, aryle, alkylaryle ou arylalkyle éventuellement substitués;
$R_4$ représente un groupe alkyle, aryle, alkylaryle ou arylalkyle substitué par au moins un groupement ester;
$A^{r\ominus}$ représente un anion organique ou inorganique ;
n et r sont des nombres entiers;
m est un nombre entier valant 1, 2 ou 3 et tel que le produit m x r = n.

2 - Procédé selon la revendication 1 caractérisé en ce que le groupe $R_4$ est substitué par au moins deux groupements ester.

3 - Procédé selon l'une quelconque des revendications 1 et 2 caractérisé en ce qu'un au moins des groupes Rl, $R_2$ et $R_3$ est substitué par au moins un groupement ester.

4 - Procédé selon la revendication 3 caractérisé en ce que les trois groupes $R_1$, $R_2$ et $R_3$ sont substitués chacun par au moins un groupement ester.

5 - Procédé selon l'une quelconque des revendications 1 à 4 caractérisé en ce que les groupements ester sont des groupements acétoxy.

6 - Procédé selon l'une quelconque des revendications 1 à 4 caractérisé en ce que les groupements ester sont sélectionnés parmi les groupements ester aliphatique à chaîne droite ou ramifiée comprenant 8 à 10 atomes de carbone.

7 - Procédé selon la revendication 5 caracatérisé en ce que l'activant est sélectionné parmi les sels de trialkyl-(2,3-diacétoxy-propyl)-ammonium, les sels d'alkyl-tris(2-acétoxyéthyl)-ammonium, les sels d'alkyl-tris(2,3-diacétoxypropyl)-ammonium, les sels de tétrakis(2-acétoxyéthyl)-ammonium et les sels de (2,3-diacétoxy-propyl)-tris(2-acétoxyéthyl)-ammonium.

8 - Procédé selon l'une quelconque des revendications 1 à 7 caractérisé en ce que le peroxyde d'hydrogène est généré in situ dans le bain de lavage par la mise en solution d'un persel.

9 - Procédé selon la revendication 8 caractérisé en ce que le persel est sélectionné parmi les perborates et les percarbonates des métaux alcalins.

10 - Compositions solides de lavage ou de désinfection comprenant un composé susceptible de générer du peroxyde d'hydrogène en solution aqueuse caractérisées en ce qu'elles contiennent un activant conforme à celui utilisé dans le procédé selon l'une quelconque des revendications 1 à 9.

11 - Utilisation des compositions selon la revendication 10 dans des bains pour le lavage et la désinfection de textiles et de surfaces dures.

Office européen des brevets

# RAPPORT DE RECHERCHE EUROPEENNE

0186052

Numero de la demande

EP 85 11 5809

## DOCUMENTS CONSIDERES COMME PERTINENTS

| Catégorie | Citation du document avec indication, en cas de besoin des parties pertinentes | Revendication concernee | CLASSEMENT DE LA DEMANDE (Int Cl 4) |
|---|---|---|---|
| D,A | GB-A-1 382 594 (UNILEVER) * Page 1, ligne 13 - page 2, ligne 15 * | 1,8-11 | A 01 N 59/14<br>A 01 N 59/00<br>C 11 D 3/39 //<br>(A 01 N 59/14<br>A 01 N 37:02 )<br>(A 01 N 59/00<br>A 01 N 37:02 ) |
| A | FR-A-2 353 634 (PROD. CHIM. UGINE KUHLMANN) * En entier * | 1,3-11 | |
| A | FR-A-2 256 279 (HOECHST) * Revendications * | 1,10, 11 | |
| A | CHEMICAL ABSTRACTS, vol. 98, no. 11, 14 mars 1983, page 180, résumé no. 84788x, Columbus, Ohio, US; M. RUCKA et al.: "New biocides for cooling water treatment. I. Selected quarternary ammonium salts" & ENVIRON. PROT. ENG. 1980 (PUB. 1981), 6(4), 455-64 | 1,11 | DOMAINES TECHNIQUES RECHERCHES (Int Cl 4)<br>A 01 N<br>C 11 D |

Le present rapport de recherche a été etabli pour toutes les revendications

| Lieu de la recherche | Date d achevement de la recherche | Examinateur |
|---|---|---|
| LA HAYE | 19-03-1986 | FLETCHER A.S. |

CATEGORIE DES DOCUMENTS CITES

X particulièrement pertinent à lui seul
Y : particulièrement pertinent en combinaison avec un autre document de la même catégorie
A : arrière-plan technologique
O : divulgation non-écrite
P : document intercalaire

T théorie ou principe à la base de l invention
E : document de brevet antérieur, mais publié à la date de dépôt ou apres cette date
D : cité dans la demande
L : cité pour d'autres raisons
& membre de la même famille document correspondant

OEB Form 1503 03 82